# EUROPEAN PATENT APPLICATION

(11) **EP 1 002 532 A1**
(43) Date of publication of application: **24.05.2000**
(21) Application number: 98203923.2
(22) Date of filing: 19.11.1998
(51) Int. Cl.: A61K 31/195

(54) **Prevention of aging effects and treatment of muscle atrophy**

(71) Applicant: K.U. Leuven Research & Development, 3000 Leuven (BE)
(72) Inventor: Hespel, Peter Jozef Leo, 3050 Oud-Heverlee (BE)
(74) Representative: Van Someren, Petronella F. H. M.

(57) **Abstract**

The present invention relates to the use of a creatine compound, in particular creatine or a creatine analogue for the manufacture of a therapeutic preparation for reducing the biological process of aging, for treating disuse muscle atrophy or for stimulating subsequent restoral of muscle mass in rehabilitation training. The muscle atrophy is the result of immobilisation, or reduced level of physical activity due to either disease or aging. The biological process of aging comprises for example the simultaneous degeneration and degradation of body tissues, including muscle and bone, neural tissue and secretory cells. The invention further relates to a therapeutic preparation for treating or preventing disuse muscle atrophy and for inhibiting the biological process of aging, comprising a suitable carrier, diluent or excipient and an effective amount of one or more creatine compounds.

## Description

The present invention relates to the new use of creatine compounds and for the prevention of effects associated with aging and in the treatment of disuse atrophy.

Oral supplementation of healthy individuals, in particular athletes, with creatine compounds, such as creatine and creatine derivatives, has been shown to improve performance during high intensity intermittent exercise. However, no beneficial effects are observed in the case of single bouts of various modes of exercise. Although longterm creatine loading has rapidly developed as a standard ergogenic practice for athletes, there was initially no scientific evidence that this practice could be in fact beneficial.

Disuse atrophy is a degeneration of muscle tissue due to immobilisation. Such atrophy can for example result when a broken extremity like a leg or an arm is immobilised in a cast for some weeks, or when a patient is forced to bedrest due to disease. The atrophy is then usually clearly visible by a slimming of the leg or arm.

It is the object of the invention to provide a therapeutic preparation for treating or preventing muscle atrophy caused by disuse.

According to the invention it has now been found that a relatively low dosage of one or more creatine compounds can reduce muscle disuse atrophy. Thus, by administering to a subject who is under the risk of developing muscle atrophy, a suitable amount of one or more creatine compounds the atrophy can be reduced or even avoided. The treatment can be a preventive treatment when the therapeutic preparation is administered to the subject from the onset of the risk to develop an atrophy. Also, the preparation can be given to patients already suffering from muscle atrophy. Given that the observed anabolic action of creatine presumably is due to stimulation of secretion of anabolic hormones, including growth hormone, testosteron and insulin, creatine can also be used for any indication related to a deficiency in the secretion of those hormones.

For the effective treatment of muscle atrophy the creatine compound is to be administered on a daily basis, or on an intermittent basis in a total daily amount of between 1 and 5 g per day. This amount can also be given in more than one portion over the day. The one or more creatine compounds can be combined with suitable excipients, diluents, carriers etc. to obtain a dosage form for administration. Suitable dosage forms are drinks, tablets-capsules, powders, sweets or any nutritional supplement.

Surprisingly, it has further been found that the administration of one or more creatine compounds can lead to an improved glucose tolerance.

This led the present inventors to the finding that creatine treatment can also be used in elderly to reduce the physical effects resulting from the biological process associated with aging. Especially the combination of muscle atrophy reduction, improvement of glucose tolerance and inhibition of the degeneration of neural tissue is very useful for treating or preventing various symptoms related to aging, such as the degeneration and degradation of body tissues, including muscle and bone, neural tissue and secretory cells.

According to a further aspect thereof, the invention thus relates to the use of one or more creatine compounds for inhibiting the biological process associated with aging.

In this application the creatine compound is preferably administered in an amount that compensates the daily loss that is excreted in the urine. This amount is usually 1 to 2.5 g per day. The dosage forms described above can also be used in this application. The term "therapeutical preparation" is used in this application to encompass both preparations for treatment, i.e. drugs, and nutritional supplements for example in the form of food stuffs in liquid or solid form that contain additional creatine.

The present invention will be further illustrated in the examples that follow. In the examples reference is made to the following figures:

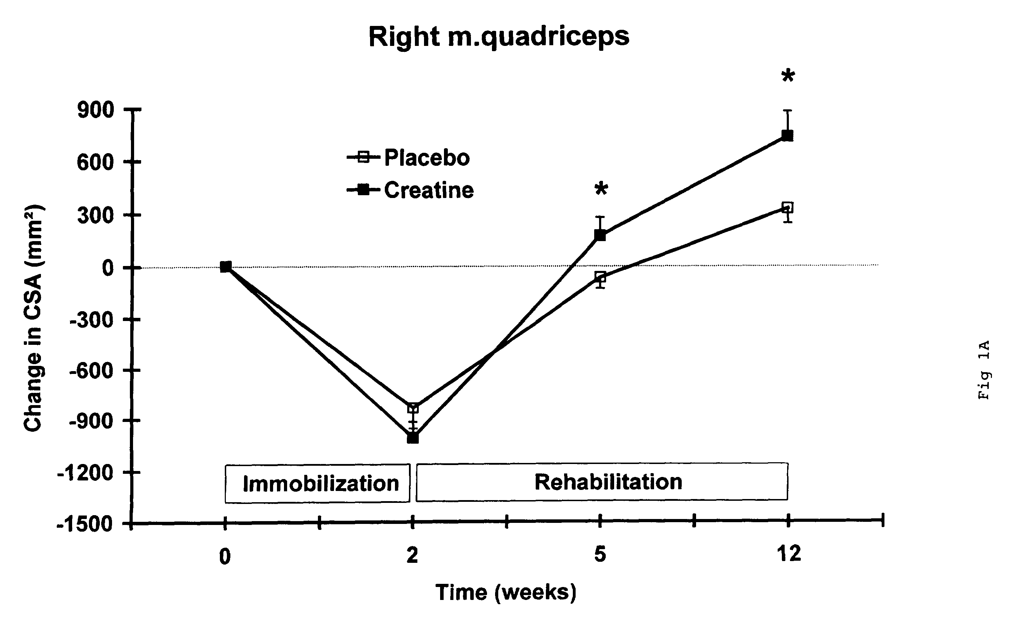

Figure 1: Effect of oral creatine intake on m. quadriceps cross-sectional area during leg immobilization and rehabilitation. Values are mean ±SEM (N=10) and represent the change of muscle CSA compared with baseline which was set equal to zero. A cast first immobilized the right leg (panel A) during a period of 2 weeks. Thereafter subjects participated in a 10 weeks rehabilitation program for the knee extensors of only the right leg. The left leg (panel B) meanwhile served as a control leg. M. quadriceps CSA was measured by NMR imaging. * Refers to a significant treatment effect compared with placebo, P<0.05.

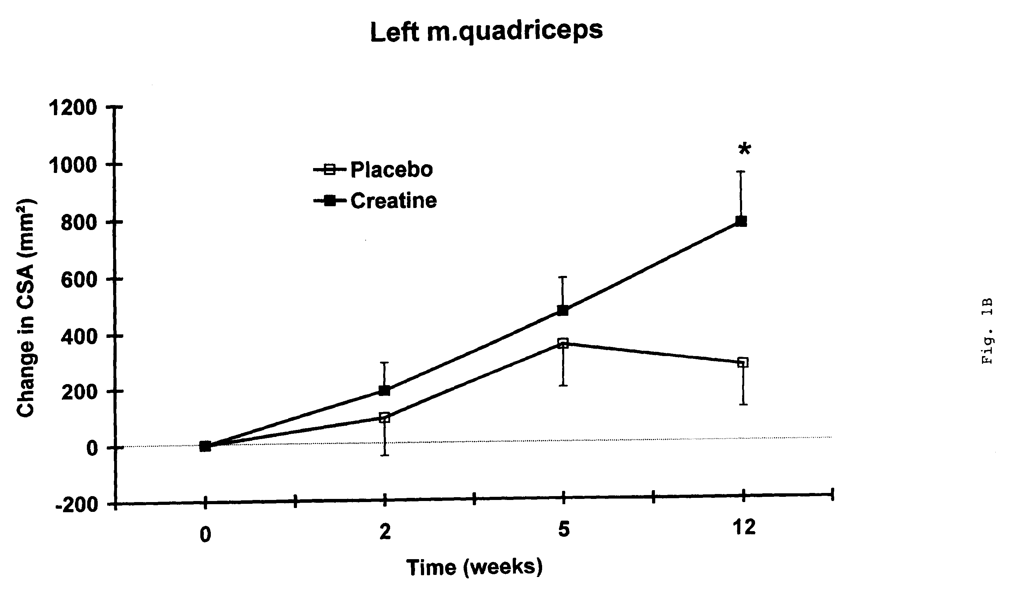

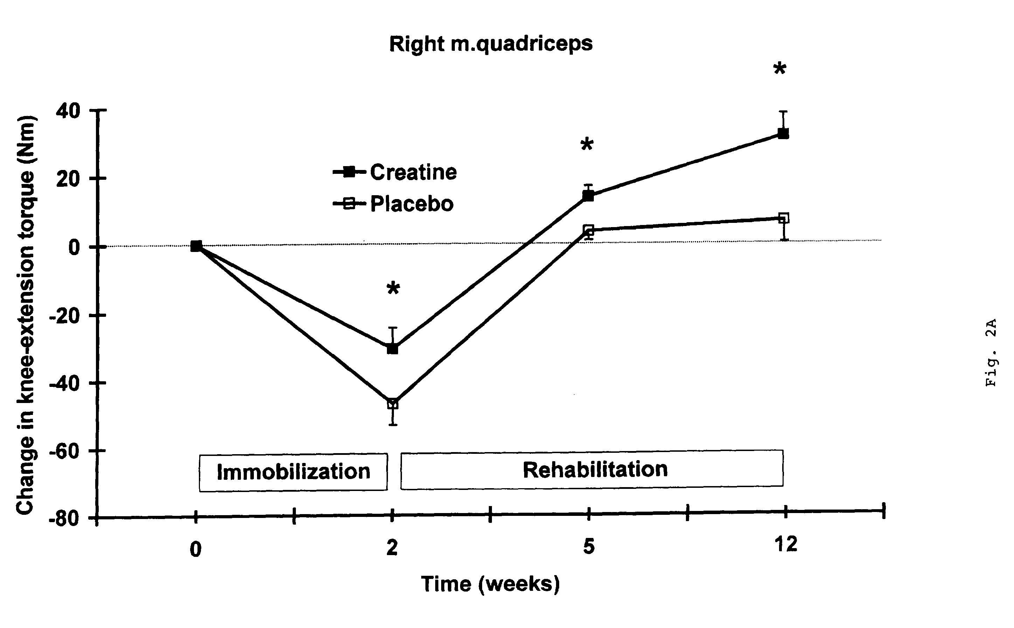

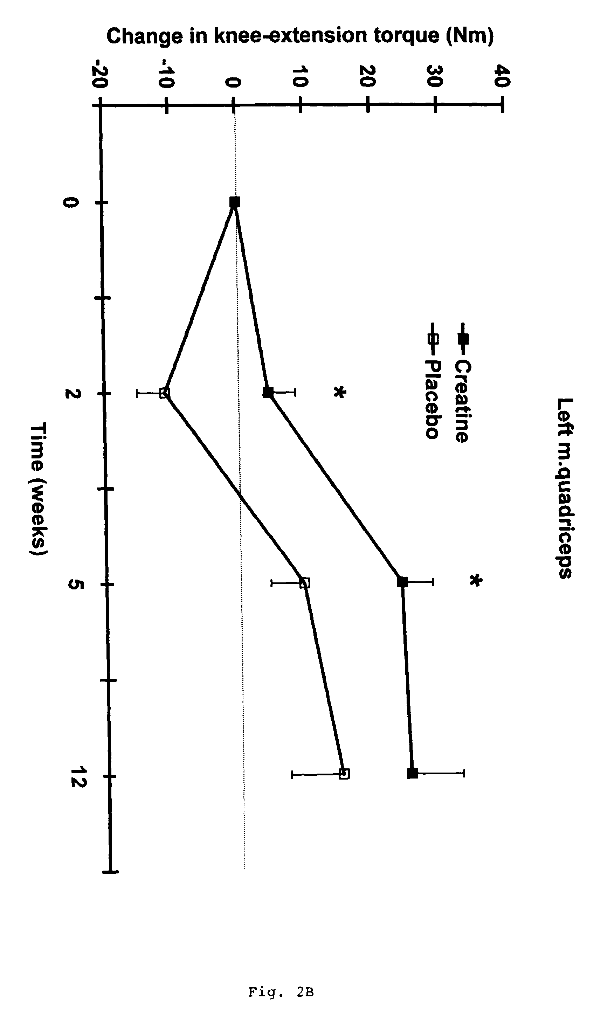

Figure 2: Effect of oral creatine intake on maximal isometric knee-extension torque during leg immobilization and rehabilitation. Values are mean ±SEM (N=10) and represent the change of maximal isometric knee-extension torque at a knee angle of 110° compared with baseline which was set equal to zero. A cast first immobilized the right leg (panel A) during a period of 2 weeks. Thereafter subjects participated in a 10 weeks rehabilitation program for the knee extensors of only the right leg. The left leg (panel B) meanwhile served as a control leg. Knee-extension torque was measured by an isokinetic dynamometer. * Refers to a significant treatment effect compared with placebo, P<0.05.

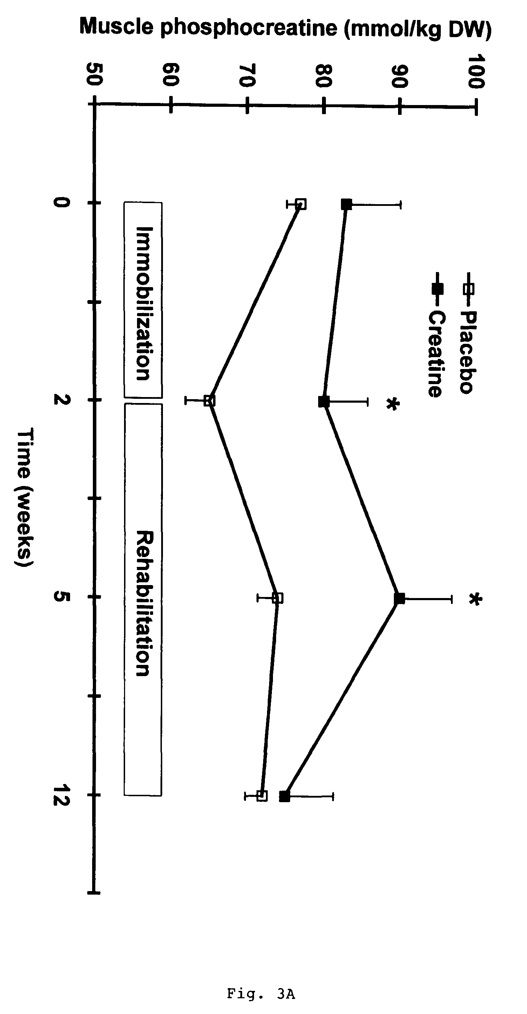

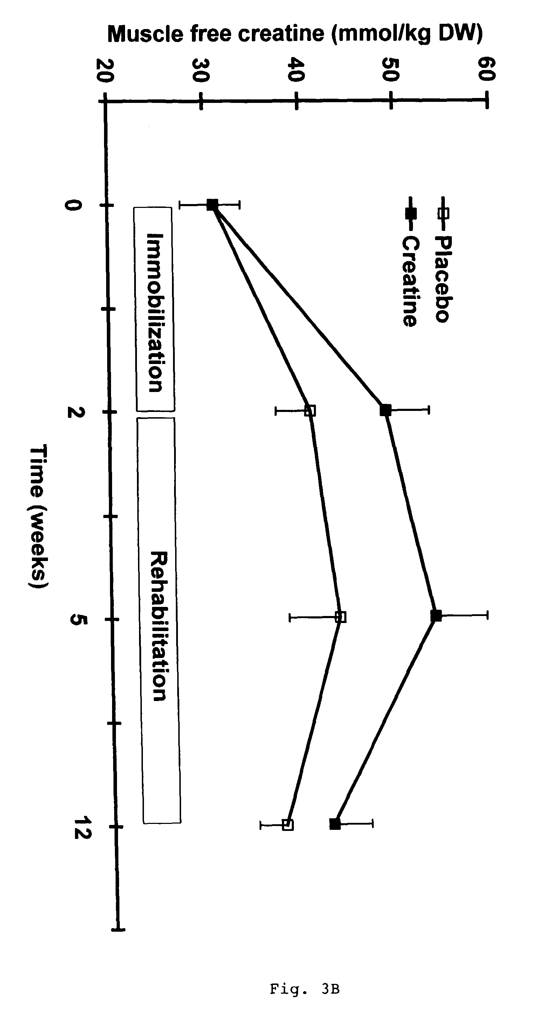

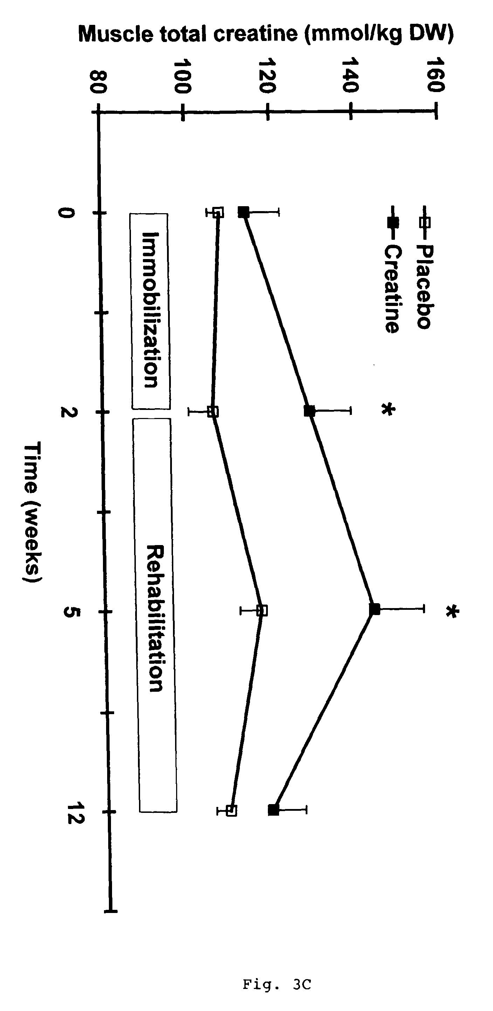

Figure 3: Effect of oral creatine supplementation on muscle creatine content during leg immobilization and rehabilitation. Values are mean ±SEM (N=10). Needle biopsy samples were obtained from the right m. vastus lateralis. Muscle phosphocreatine (panel A), free creatine (panel B) and total creatine (panel C) concentrations are shown. Muscle total creatine concentration was calculated as the sum of muscle phosphocreatine and free creatine concentrations. A cast first immobilized the leg during a period of 2 weeks. Thereafter subjects participated in a 10 weeks rehabilitation program for the knee-extensors. * Refers to a significant treatment effect compared with placebo, P<0.05.

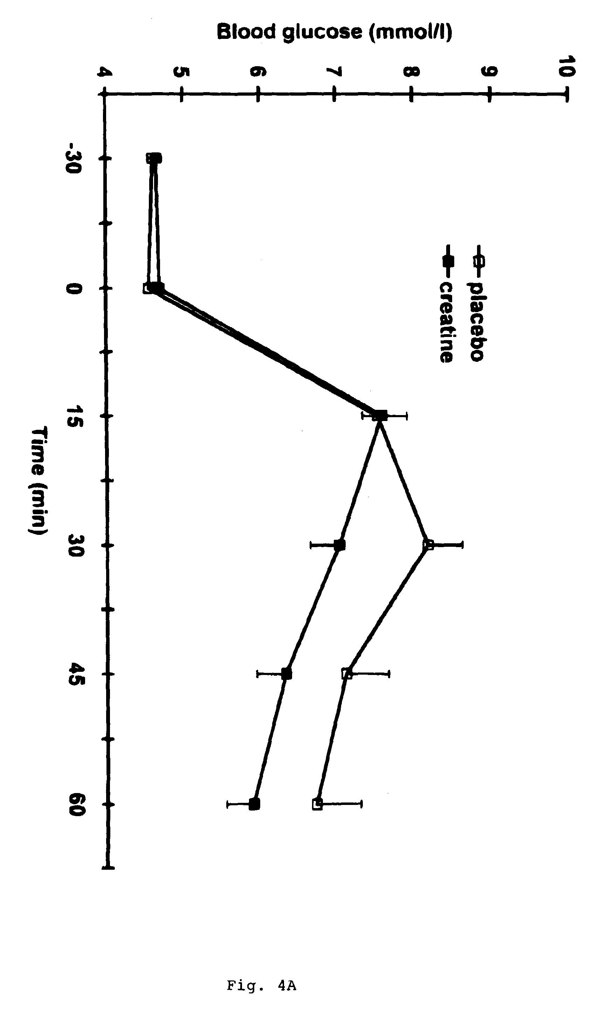

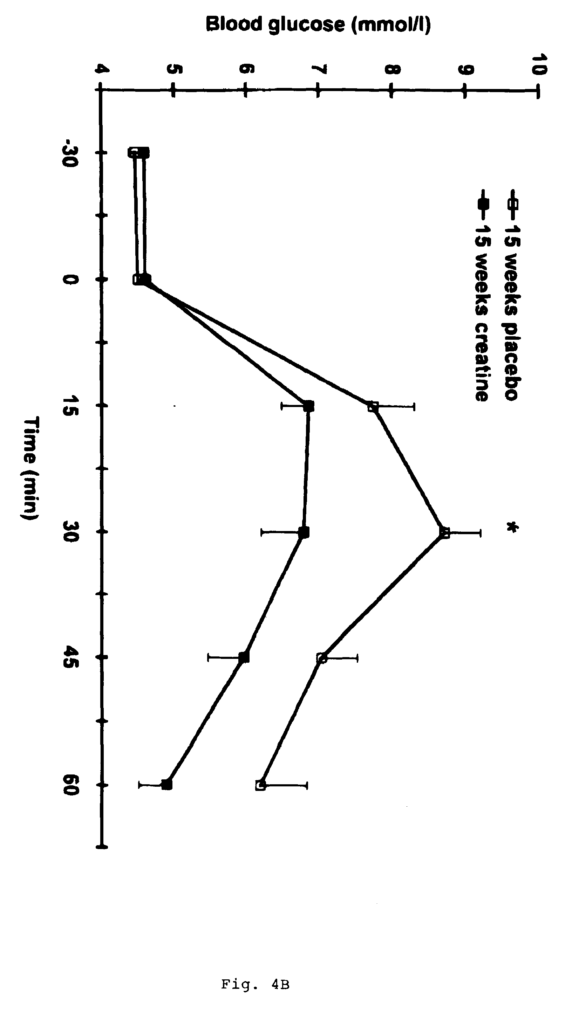

Figure 4: Effect of accute and long-term creatine intake on the response of blood glucose concentration to oral glucose intake. Values are means ±SEM of 8-9 observations. Subjects received 1g of glucose per kg BW at time t₀, both before (panel A) and after (panel B) 15 weeks of either oral creatine monohydrate supplementation or placebo. Thirty min before the glucose intake (t₃₀) subjects of the creatine group ingested 10 g of creatine monohydrate, versus a placebo in the placebo group. Blood glucose concentration was measured every 15 min on capillary blood samples from the hyperaemic earlobe. * denotes a significant difference (p<0.05) compared with the corresponding placebo value.

### EXAMPLES

### EXAMPLE 1

### Use of creatine for the treatment of disuse atrophy

### 1. Materials and Methods

### 1.1 Subjects

Twenty healthy subjects, 10 males and 10 females, ranging in age from 20 to 23 years gave their informed written consent to take part in the study. They were informed in detail of all experimental procedures to be undertaken and were asked to abstain from any medication during the period of the study and to avoid changes in their diet or level of physical activity. Three of the female subjects were taking oral contraceptive medication.

### 1.2 Study Protocol

A double-blind study was performed, during the preparation of which subjects reported twice to the laboratory to become familiar with the isokinetic muscle force tests to be undertaken. During the first week of the study baseline measurements were performed (Session 1, week 0; see figure 1). On day 1 and after a light standardized meal (600 kcal, 60% carbohydrates, 25% fat, 15% proteins) m. quadriceps cross-sectional area (CSA) was measured by Magnetic Resonance Imaging (MRI), where after a percutaneous needle biopsy of the right vastus lateralis was taken for biochemical and histochemical analyses. On day 3 subjects collected a 24-hour urine sample. Finally, on day 4 and again after a light standardized meal isometric and dynamic maximal knee-extension torque of the right and left leg were evaluated using an isokinetic dynamometer. Subsequently subjects were assigned to either a creatine (CR: n = 10) or a placebo (P: n = 10) group so as to obtain two groups of similar distribution for sex, and cross-sectional area of m. quadriceps. The CR group received 5 g of creatine monohydrate that was flavored by the addition of citrate (60 mg(per g creatine) and maltodextrine (490 mg per g creatine), 4 times per day, while the P group received placebo supplements (5g maltodextrine containing 40 mg(g⁻¹ citrate) 4 times per day. Subjects were instructed to dissolve the supplements in hot water within 1 min before intake. Creatine and placebo powders were identical in taste and appearance. The subjects' right leg was then immobilized at a knee angle of ^{∼}160° by a light polyester cast, reaching from groin to ankle. Subjects received crutches and free access to private transportation services in order to limit loading of the immobilized leg.

During the 5th day of immobilization subjects collected a 24-hour urine sample. Two days later the cast was removed and the knee-joint was mobilized by passive exercises for 20 min, after which subjects were allowed to take a shower. Immediately after a new cast was adapted for another week of immobilization. At the end of the second week of immobilization the cast was removed and post-immobilization measurements were performed (Session 2, Week 2).

Session 2 was identical to session 1, yet on the 1st to 3rd day following removal of the cast subjects participated in a 30 min physiotherapy session (passive mobilization) aiming to restore normal knee-joint mobility before the assessment of maximal knee extension torque on day 4. Immediately after session 2, a 10 weeks rehabilitation program was started. Subjects participated in a unilateral training program for the right leg, at a rate of 3 sessions per week. Each training session consisted of 4 series of 12 unilateral knee-extensions (Technogym®) ranging from a 90° knee-angle to full extension at a rate of 60°sec⁻¹. Workload was set at 60% of maximum isometric knee-extension torque, which was measured at a 90° knee-angle at the start of each session using a calibrated isometric muscle tension transducer.

During the later 7 weeks of the training period, 6 instead of 4 contraction series were performed. All training sessions were supervised by one of the investigators. The CR/P dosage was reduced from 4 times 5g per day during immobilization to 3 times 5g per day during the initial 3 weeks of rehabilitation, and further to a single 5g dosage daily thereafter.

After 3 (Session 3, Week 5) and 10 (Session 4, Week 12) weeks of rehabilitation, and at least 48 hours following the last training session, subjects returned to the laboratory for an intermediate and a post-training evaluation session, respectively. All measurements done through sessions 1 to 4 were for each subject done on the same day of the week and at the same time of the day.

The 10-weeks washout period was assumed to be sufficient so as to allow normalization of body creatine stores following the prior 12 weeks creatine loading period.

The serum samples that originally were assayed for creatine, glucose, insulin, growth hormone and IGF-1 concentrations.

### 1.3 Determination of quadriceps muscle cross-sectional area

NMR imaging was performed in a 1.5 T scanner (Vision, Siemens) using a phased array body coil positioned over the upper legs. Subjects were lying on the scanner table that was instrumented with a plastic leg holder allowing accurate and reproducible positioning of the subjects' legs with reference to the coil and magnet during the different MRI sessions. T1 weighted images were acquired with a spin-echo sequence (TR/TE=500/12 ms). First the most distal point of the medial condylus of the femur was located by frontal scanning. Thereafter three axial slices with 10 mm thickness and 30 mm spacing, were positioned on coronal slices at 17, 20 and 23 cm proximal to the reference point. The in-plane resolution of the axial images was 1.12x0.78 mm. Images were transferred to a dedicated workstation for quantitative processing. CSA of mean quadriceps was determined by digitization of the images using Visual Basic software (Microsoft Co., USA). Digitization was done in duplicate by two independent investigators, after which values were averaged. However, if either intra-observer or inter-observer differences for a given image exceeded 5%, the investigator re-digitized the image. For each single image CSA was eventually calculated as the mean of the values obtained by the 2 investigators. Finally, quadriceps CSA was expressed as the mean (mm2) of the 3 axial scan images taken at 17, 20 and 23 cm proximal to the medial condylus of the femur, respectively.

### 1.4 Determination of knee-extension torque

Maximal torque and fatigue of the knee-extensors was evaluated on an isokinetic dynamometer that was calibrated prior to each experiment. The self-constructed dynamometer was made of a computer controlled asynchronous electromotor (AMK Dynasyn, 19kW), instrumented with a torque transducer (Lebow, maximal torque 565 Nm, 0.05% precision). The exercise test consisted of unilateral knee-extensions performed in a sitting position on the dynamometer.

After a 5-min standardized warming-up, the subjects performed 3 maximal voluntary isometric contractions (5 sec), interspersed by 2 min rest intervals, at a knee-angle of 110°. Maximal isometric torque was then derived as the smoothed curve during the static contraction.

After a 10 min rest-interval subjects performed 3 bouts of 30 dynamic maximal voluntary contractions separated by 2-min rest intervals. Knee-extension torque was measured during each contraction and digitized (250 Hz) by an on-line computer. The dynamic torque was calculated as the mean torque during maximal knee-extension at a constant velocity of 180°·sec-1, starting from 90° to full extension (180°). After each contraction the leg was returned (180°·sec-1) passively to the starting position from which the next contraction was immediately initiated. Torque production was registered as the mean of five successive contractions.

### 1.5 Muscle biochemistry and histochemistry

Muscle samples were obtained from vastus lateralis of the right leg by using the needle biopsy technique (Bergström, 1962) with suction being applied. Incisions were made through the skin and muscle fascia under local anesthetic (2-3 ml of 1% lidocaine). During sessions 2, 3 and 4 the incision was made either proximal or lateral to the incision made at an earlier session. Needle biopsy samples were immediately blotted and cleaned of connective tissue. The remaining part of the muscle sample was frozen in liquid nitrogen and stored at -80°C until biochemical analyses were performed at a later date.

Muscle samples were freeze dried and washed twice in petroleum ether to remove fat. Thereafter a portion of each sample was dissected free of visible blood and connective tissue and was pulverized. The powdered extract was then used for spectrophotometric determination of ATP, PCr and free Cr concentrations (Harris et al., 1974). Muscle total creatine concentration was calculated as the sum of PCr and free Cr concentrations.

### 1.6 Urine and plasma biochemistry

Urine samples were thoroughly mixed and transferred to a graduated cylinder for determination of 24 h urine volume. Two 25 ml aliquots were separated and frozen at -20°C until analyzed at a later data. Urinary creatine and creatine concentrations were measured by HPLC as previously described (Green et al, 1996). Twenty-four hour urinary creatine and creatinine excretions were calculated by multiplying urinary creatine and creatinine concentrations by the corresponding 24h urinary output.

Blood samples were collected into sterile vacuum tubes (Vacutainer®) containing clot activator. Samples were kept on ice until centrifuged for separation of serum within 2 hours. Serum was stored at -20°C until analyzed at a later date. Serum creatine concentration was determined using a standard enzymatic fluorometric assay (Bergmeyer, 1985).

### 1.7 Statistical analysis

All data are expressed as mean ± SEM. Statistical evaluation (Statistica® software, Ohio, USA) of the data was performed using unpaired t-test and repeated measures two-way analysis of variance, using Sheffe's test for post-hoc multiple comparisons where appropriate. The level of statistical significance was set at p<0.05.

### 2. Results

### 2.1 Side-effects and treatment identification

At the end of the study the subjects were asked as to whether they had any notion of the treatment received. After either creatine or placebo treatment all subjects reported unsure about the treatment. No side-effects were spontaneously reported during the entire duration of the study.

### 2.2 Muscle cross-sectional area

In the right leg at baseline m. quadriceps cross-sectional area (CSA) was 9.0 ± 0.5 cm² in P versus 9.2 ± 0.5 cm2 in CR (N.S.). As shown in figure 1 (panel A), during the 2 weeks of casting quadriceps CSA decreased (p<0.05) by ∼10% in both groups. During subsequent rehabilitation quadriceps CSA rapidly reverted to normal. However, the increase of muscle CSA occurred at a faster (p<0.05) rate in CR than in P. Compared with post-immobilization CSA, after 3 weeks of rehabilitation the increment in quadriceps CSA amounted to 14% in CR versus 9% in P (p<0.05). Accordingly, after 10 weeks the gain of quadriceps CSA was 14% in P versus 21% in CR (p<0.05). Thus compared with baseline values, at the end of the rehabilitation period right m. quadriceps CSA was greater (p<0.05) in CR (10.0 ±0.6) but not in P (9.3 ± 0.4 cm²).

In the left leg, at the start of the study quadriceps CSA was 9.2 ± 0.4 cm² in P and similar in CR (9.3 ± 0.5 cm²). As shown in figure 1 (panel B) in P quadriceps CSA did not significantly change throughout the study. Conversely, in CR CSA progressively increased to a value which at week 12 (10.0 ± 0.6) was higher (p<0.05) than at baseline.

### 2.3 Muscle performance

### 2.3.1 Maximal isometric knee-extension torque

Compared with P (182 ± 13 Nm), at the start of the study maximal isometric knee-extension torque of the right leg was slightly lower (N.S.) in CR (165 ± 10 Nm). As shown in figure 2, during immobilization torque decreased in both groups. However, the torque regression produced by casting was smaller (p<0.05) in CR (-30 ± 6 Nm) than in p (-47 ± 6 Nm). During rehabilitation torque markedly increased (p<0.05) in either group. At the end of the rehabilitation period torque had just returned to baseline in P. Conversely, in CR knee-extension torque at the end of the study was higher (p<0.05) than at baseline. In the left leg, in P maximal torque did not significantly change throughout the study. In contrast, in CR a progressive gain (p<0.05) of knee-extension torque was noted.

### 2.4 Muscle metabolites

Muscle biopsies were taken from vastus lateralis of the right leg only. Muscle ATP concentration ranged from 18.1 ±0.5 to 21.5 ± 0.5 mmol.kg⁻¹ Dry Weight (D.W.) and was not significantly affected by immobilization and rehabilitation in either experimental group. As shown in figure 3, muscle phosphocreatine (PCr) concentration at baseline was not significantly different between groups. During the 2 weeks immobilization period muscle PCr markedly decreased (p<0.05) in P, yet not in CR. Hence compared with P, after casting muscle PCr level was higher (p<0.05) in CR. During the initial 3 weeks of rehabilitation muscle PCr increased at similar rate in both groups. Thus compared with P, PCr concentration remained elevated (p<0.05) However, due to falling muscle PCr level in CR from the 3rd to the 10th week of rehabilitation, at the end of the rehabilitation period muscle PCr had returned to similar and normal baseline values in either group. No significant differences (p<0.10) were noted for muscle free creatine concentration between P and CR either at baseline or during and after immobilization. Muscle total creatine content was similar at baseline for the 2 experimental groups. However, both after immobilization and after 3 weeks of rehabilitation muscle total creatine level was higher (p<0.05) in CR than in P. However, by the end of the 10 weeks rehabilitation period this difference was abolished.

### EXAMPLE 2

### Effect of creatine administration on glucose tolerance

Because the present inventors contemplated that creatine intake possibly could stimulate insulin action on skeletal muscle an oral glucose tolerance test was added to the study protocol.

### Methods

At the end of the 10-week training period, and at least 48 hours following the last training session, 17 subjects (PL, N = 8; Cr, N = 9) reported to the laboratory in the morning after an overnight fast. Subjects were seated in a comfortable chair and remained in the seated position during the entire experiment. After 15 min of rest at -30 min (t₋₃₀) a 75 µl capillary blood sample was taken from the hyperaemic (Finalgon®) earlobe using Na-heparinized glass capillaries. Immediately after a 10 ml blood sample was taken from an antecubital vein into Na-heparinized tubes (Vacutainer®). Subjects from the Cr group were then administered with 10 g of creatine monohydrate which was dissolved in 100 ml of sweetened (aspartame) water. PL subjects received a similar volume of sweetened water. Thirty min later (t₀) a second capillary blood sample was taken, whereupon the subjects ingested 1 g of glucose per kg BW, which was dissolved in 300 ml of cold water. At t₁₅, t₃₀, t₄₅, t₆₀ capillary blood samples were taken. Furthermore, another blood sample from an antecubital vein was taken at t₃₀. Throughout this manuscript data obtained during this experimental session will be referred to as the creatine loaded condition.

Thereafter creatine and placebo supplementation was stopped for 10 weeks. Such washout period conceivably is sufficient to allow normalisation of body creatine stores following the prior 12 weeks creatine loading period. In this respect, we recently have demonstrated muscle phosphocreatine concentration to regress to normal within 5 weeks following cessation of high dose creatine intake for 20 weeks (Vandenberghe, 1997). Subjects reported back to the laboratory on the same day of the week and time of the day, and underwent an oral glucose tolerance test identical to 10 weeks before. Measurements obtained in this session are referred to as baseline throughout this manuscript.

Capillary blood samples were immediately analysed for glucose concentration using an automated glucose analyser (Y.S.I.glucose analyser, model 2300 STAT). Thereafter, plasma was separated immediately by high-speed centrifugation and was stored at -80°C until assayed for creatine and insulin concentration. Creatine was measured using a standard enzymatic fluorometric assay (Bergmeyer, 1985). Insulin was determined by a double-antibody radio-immunoassay with rat insulin as the standard (Novo Research Institute, Bagsvaerd, Denmark).

Blood glucose disposal was defined as the area under the Δblood-glucose curve (see figure X) from 0 to 60 min after glucose ingestion. The glucose area (GA) was calculated according to the trapezoidal rule. All data are given as means ± SEM. Differences between experimental groups were statistically evaluated by either unpaired t-test or repeated measures two-way-analysis of variance where appropriate, using Statistica software (Statsoft Inc., Tulsa, USA). A probability level of p<0.05 was set as the criterion for statistical significance.

### Results

At baseline blood glucose concentration in P increased from 4.6 + 0.1 mmol·l⁻¹ before (t₀) to a peak value reaching 8.2 ± 0.4 mmol·l⁻¹ 30 min (t₃₀) after the glucose administration (figure 1, panel a). Thereafter, blood glucose returned to 6.7 ± 0.6 mmol l⁻¹ at t₆₀. Compared with P, initial blood glucose level (t₀) at baseline was similar in CR (4.6 ± 0.1 mmol·l⁻¹) and was not acutely affected by the ingestion of a 10 g creatine dosage. Upon glucose intake blood glucose concentration peaked at t₁₅ (7.6 ± 2 mmol·l⁻¹) where after it decreased to 5.9 ± 0.5 mmol·l⁻¹ within 60 min (t₆₀). Compared with placebo, except at t₁₅, blood glucose concentration was lower in CR after glucose administration. Thus, GA was 150 ± 17 mmol·l⁻¹·min⁻¹ in P versus 114 ± 14 mmol·l⁻¹·min⁻¹ in CR (p=0.10).

At the end of 15 weeks of placebo administration in P, the response of the blood glucose concentration (GA = 164 ± 17 mmol·l⁻¹·min⁻¹) to glucose ingestion was similar to baseline (figure 1, panel b). However, in CR blood glucose concentration and GA (90 ± 33 mmol·l⁻¹·min⁻¹) tended (p=0.6) to be lower due to the 15 weeks of creatine supplementation. Thus, compared with P, in CR blood glucose level during the glucose tolerance test was markedly lower. Furthermore, similar to before creatine loading, after creatine loading acute creatine intake did not change blood glucose concentration.

At baseline, plasma insulin was similar in P (0.7±0.1 ng/ml) and CR (0.7±0.1 ng/ml). Glucose intake markedly increased plasma insulin (2.9±0.5 in CR vs. 3.0±0.4 in P). However, the impact of glucose intake on plasma insulin was independent of acute creatine intake or prior creatine long-term creatine loading.

## Claims

1. Use of a creatine compound, in particular creatine or a creatine analogue for the manufacture of a therapeutic preparation for reducing the biological process of aging.

2. Use of a creatine compound, in particular creatine or a creatine analogue for the manufacture of a therapeutic preparation for treating disuse muscle atrophy.

3. Use according to claim 2, wherein the therapeutic preparation is further intended for stimulating subsequent restoral of muscle mass in rehabilitation training.

4. Use as claimed in claims 1, 2 and 3, **characterised in that** the therapeutic preparation is intended to be administered in an amount that leads to a total daily supplementation of 0,5 to 5 g creatine.

5. Use as claimed in claim 2 or 3, **characterised in that** the muscle atrophy is the result of immobilisation, or reduced level of physical activity due to either disease or aging.

6. Use as claimed in claim 1, **characterised in that** the biological process of aging comprises the simultaneous degeneration and degradation of body tissues, including muscle and bone, neural tissue and secretory cells.

7. Therapeutic preparation for treating or preventing disuse muscle atrophy and for inhibiting the biological process of aging, comprising a suitable carrier, diluent or excipient and an effective amount of one or more creatine compounds.

8. Therapeutic preparation according to claim 7, which is a drug.

9. Therapeutic preparation according to claim 7, which is a nutritional supplement.

10. Therapeutic preparation according to claim 7 or 9, which has the form of a food stuff comprising one or more additional creatine compounds.
